# EUROPEAN PATENT SPECIFICATION

(11) **EP 4 547 341 B1**
(45) Date of publication and mention of the grant of the patent: **04.03.2026**
(21) Application number: 23731611.2
(22) Date of filing: 07.06.2023
(51) Int. Cl.: A61Q 5/02, A61Q 19/10, A61K 8/46, A61K 8/44

(54) **REDUCED PALM KERNEL RELIANT WASH COMPOSITION**
WASCHZUSAMMENSETZUNG MIT REDUZIERTER PALMKERNABHÄNGIGKEIT
COMPOSITION DE LAVAGE À BASE DE NOYAUX DE PALMIER RÉDUITS

(30) Priority: 30.06.2022 US 202263357362 P; 06.09.2022 EP 22194211
(43) Date of publication of application: 07.05.2025
(73) Proprietor: Unilever IP Holdings B.V., 6708 WH Wageningen (NL); Unilever Global IP Limited, Wirral, Merseyside CH62 4ZD (GB)
(72) Inventor: YUAN, Mingjun, 6708 WH Wageningen (NL); VASUDEVAN, Tirucherai ,Varahan, 6708 WH Wageningen (NL)
(74) Representative: Askew, Sarah Elizabeth
(86) International application number: PCT/EP2023/065241
(87) International publication number: WO 2024/002645

(56) References cited:
- EP-B1- 3 621 582
- US-A1- 2020 253 849
- US-A1- 2021 015 728
- US-A1- 2021 401 716

## Description

### Field of the Invention

The present invention is directed to a wash composition having consumer desirable characteristics where the compositions can be isotropic or lamellar. More particularly, the wash composition of the present invention is substantially free of sulfates, comprises glutamate, aspartate or both, and optionally, at least one additional anionic surfactant selected from isethionates, taurates and/or glycinates. Total anionic surfactant used in the wash composition is from 1 to 9% by weight whereby the wash composition is mild, stable, lathers well and produces a soft and smooth sensation after use. Unexpectedly, the wash composition yields such consumer desirable characteristics even when formulated with reduced levels of palm kernel oil derived surfactants.

### Background of the Invention

Consumers often seek low sulfate or even sulfate free wash compositions since such compositions, in addition to being milder on the environment, are also milder on the surfaces being cleaned, like, for example, textiles, skin and hair. Unfortunately, mild wash compositions when combined with water and shearing are typically poor at delivering consumer desirable characteristics, like desirable lather. Wash compositions that yield desirable lather are enjoyed by consumers since they generally wash longer and more when product lathering is good. During the cold and flu season, and especially during epidemics and pandemics, longer washing times are encouraged to better sanitize the surface targeted for cleaning. Also, desirable lathers elevate dirt and microbes, resulting in cleaner surfaces that are easier to rinse with less water.

In addition to having low or even no sulfates, it is desirable to reduce the amount of surfactant having hydrophobic portions derived from palm kernel oil. Palm kernel oil, which predominately consist of lauric (C₁₂) and myristic (C₁₄) fatty acids, does result in surfactants (produced by esterifications of the same with hydrophilic head groups) that yield excellent lather characteristics. However, reliance on palm kernel oil is deemed to be a major contributor to deforestation, threatens biodiversity and increases greenhouse gas emissions.

It is of increasing interest to develop wash compositions that are mild, impact the environment positively, and that simultaneously are stable and yield consumer desirable characteristics. The present invention, therefore, is directed to a wash composition that is substantially free of sulfates and has reduced levels of surfactant with hydrophobic portions derived from palm kernel oil. The wash composition of the present invention comprises glutamate, aspartate or both, and optionally, at least one additional anionic surfactant selected from isethionates, taurates and/or glycinates. Total anionic surfactant used in the wash composition typically is from 1 to 9% (or 1.8 to 8% or 2 to 7.2% or 3.3 to 7.5%) by weight whereby the wash composition surprisingly is mild, stable, produces a soft and smooth sensation after use, and unexpectedly, yields such consumer desirable characteristics even when formulated with reduced levels of surfactants that are palm kernel oil derived.

### Additional Information

Efforts have been disclosed for making wash systems. In U.S. Published Patent Application 2011/245124 A1, wash cleansers with alkanoyl compounds, fatty acyl isethionate surfactants and a skin or hair benefit agent are described.

Other efforts have been disclosed for making wash systems. In U.S. Published Patent Application 2018/263879 A1, personal care compositions with amphoacetates, glutamates and cocamide MIPA are described.

Even other efforts have been described for making wash compositions. In WO/17063806, wash compositions with alkyl ether sulfate anionic surfactant, N-acyl glutamate and an amido betaine are described.

US2021/401716 relates to sulfate free rinse-off cleansing compositions and methods of use, particularly shampoo compositions, that include high oil loads.

None of the additional information describes a wash composition as claimed herein.

### Summary of the Invention

A wash composition comprising:
a) an anionic dicarboxylate comprising a glutamate, aspartate or both, the glutamate and/or aspartate having a formula: 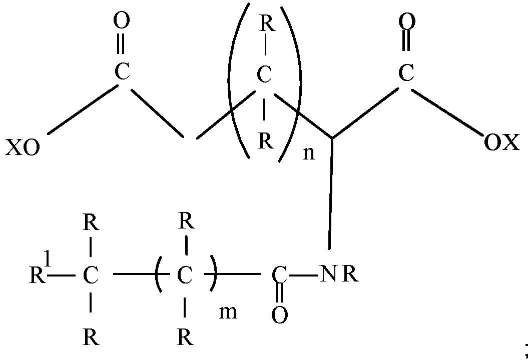
b) an amphoteric or zwitterionic surfactant or both; and
c) water

wherein total surfactant used is from 1 to 12% by weight of the composition,
wherein nonionic surfactants will make up no more than 1.5 wt%, by weight of the wash composition,
wherein the composition has a pH from 4.5 to 8.0, 1.5 to 90%, and preferably, 1.75 to 80.0% and most preferably, from 2 to 70% (or in other embodiments 25 to 55% or 35 to 45%) by weight of total weight surfactant being anionic surfactant and further wherein from 3.3 to 100%, and preferably, from 3.3 to 80%, and most preferably, from 10.0 to 45% (or in other embodiments 15 to 40% or 20 to 30% or 22 to 28%) by weight of the total weight anionic surfactant is the anionic dicarboxylate with the proviso that from 10 to 95% (or in other embodiments 50 to 90% or 65 to 85%) by weight of surfactant in the composition is neutralized,
where:
   i) each R is independently an H or a C₁₋₃ alkyl, and where at least 50% of the R groups are H, and R¹ is a C₈-C₂₂ alkyl group with the further proviso that at least 40 to 100%, most preferably, 67.5 to 100% by weight of the total weight of the anionic dicarboxylate present has a fatty acid group, R¹(CR₂)( CR₂)ₘ COO-, that is a linear C₁₆ palmitic acid group;
   ii) each X is independently H⁺, Na⁺, K⁺, Ca⁺ or Mg⁺, where each X is not simultaneously H⁺; and
   iii) n is 0 or 1, and m is an integer from 0 to 3,
the composition having at least 10 to 55% of all surfactant free of a hydrophobic group derived from palm kernel oil.

In a second aspect, the invention is directed to a wash composition comprising:
a) an anionic dicarboxylate comprising a glutamate, aspartate or both, the glutamate and/or aspartate having a formula: 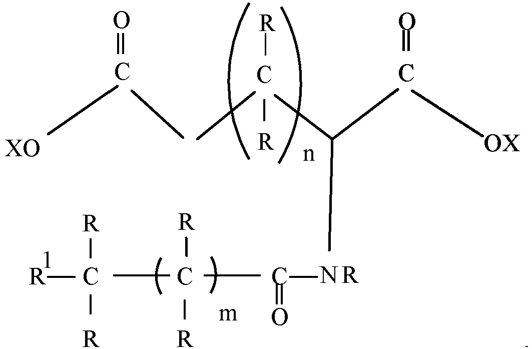
b) anionic surfactant comprising a taurate and isethionate;
c) at least one surfactant which is a zwitterionic surfactant; and
d) water

wherein total surfactant used is from 1 to 12% by weight of the composition,wherein nonionic surfactants will make up no more than 1.5 wt%, by weight of the wash composition,
wherein the composition has a pH from 4.5 to 8.0, 1.5 to 90%, and preferably, 1.75 to 80.0% and most preferably, from 2 to 70% (or in other embodiments 25 to 55% or 35 to 45%) by weight of total weight surfactant being anionic surfactant a+b and further wherein from 3.3 to 90%, and preferably, from 3.3 to 80%, and most preferably, from 10.0 to 45% (or in other embodiments 15 to 40% or 20 to 30% or 22 to 28%) by weight of the total weight anionic surfactant a+b is the anionic dicarboxylate with the proviso that from 10 to 95% (or in other embodiments 50 to 90% or 65 to 85%) by weight of surfactant in the composition is neutralized,
where:
   i) each R is independently an H or a C₁₋₃ alkyl, and where at least 50% of the R groups are H, and R¹ is a C₈-C₂₂ alkyl group with the further proviso that at least 40 to 100%, and most preferably, 67.5 to 100% by weight of the total weight of the anionic dicarboxylate present has a fatty acid group, R¹(CR₂)( CR₂)ₘ COO-, that is a linear C₁₆ palmitic acid group;
   ii) each X is independently H⁺, Na⁺, K⁺, Ca⁺ or Mg⁺, where each X is not simultaneously H⁺; and
   iii) n is 0 or 1, and m is an integer from 0 to 3,
the composition comprising:
   I. from 0.5 to 7% by weight anionic dicarboxylate comprising a glutamate, aspartate or both;
   II. from 0.5 to 6% by weight isethionate making up from 5 to 40% by weight of the total weight percent of anionic surfactant;
   III. from 0.5 to 8% by weight taurate making up from 5 to 70% by weight of the total weight percent of anionic surfactant; and
   IV. 0.5 to 8% by weight zwitterionic surfactant comprising a betaine
the composition having at least 10 to 55% of all surfactant free of a hydrophobic group derived from palm kernel oil.

In a third aspect, the invention is directed to a method for cleaning a surface comprising the steps of:
a) contacting a surface with the wash composition according to the first and/or second aspect of the invention; and
b) shearing the wash composition on the surface;
c) rinsing and/or wiping the wash composition off the surface with water.

All other aspects of the present invention will more readily become apparent from the description and examples which follow.

Skin, as used herein, is meant to include skin on the arms (including underarms), face, feet, neck, chest, hands, legs, buttocks and scalp (including hair). Wash composition as used herein includes and is not limited to home care cleaning compositions and cosmetic wash compositions. The cosmetic wash compositions include shampoos, make-up washes, facial washes, personal care liquid body washes and 2-in-1 hair and body washes. Reduced levels of surfactants that are palm kernel oil derived means from 10 to 55%, and preferably, from 15 to 45%, and most preferably, from 20 to 35% of all surfactant in the wash composition is free of a hydrophobic group derived from palm kernel oil. Crude palm oil, wheat germ oil, pumpkin seed oil, rice bran oil and olive oil may be used as sources for hydrophobic group supply.

The wash composition of the present invention is ready for topical application and to be wiped or washed off, and preferably, washed off, with water. The composition may, optionally, comprise medicinal or therapeutic agents, but preferably, is a wash which is cosmetic and non-therapeutic. In one embodiment of the invention, the composition can be a home care composition like a laundry, upholstery, glass, table-top, sink or toilet cleaning composition. In another embodiment, the composition is a shampoo composition. In still another embodiment, the wash composition is a personal wash and cleansing composition. In yet another embodiment of the invention, the wash composition is a baby wash composition. As hereinafter described, the composition of the present invention, when cosmetic, may optionally comprise skin benefit ingredients added thereto such as emollients, vitamins and/or derivatives thereof, resorcinols, retinoic acid precursors, colorants, moisturizers, sunscreens, mixtures thereof or the like. The skin benefit ingredients (or agents) may be water or oil soluble. If used, oil soluble skin benefit agents typically make up to 1.75% by weight of the wash composition whereby water-soluble skin benefit agents, when used, typically make up to 10% by weight of the composition. The wash composition typically has a pH from 4.5 to 8.0. Viscosity, unless noted otherwise, is taken with a Discovery HR-2 Rheometer using sand blasted plates with a 1,000 micron gap and a shear rate of 4-15 s⁻¹ at a temperature of 25 °C. Stable wash composition, as used herein, means no discoloration, odour, phase separation and visible precipitate seen in the composition after being stored under conditions of atmospheric pressure and for at least one (1) month at 25°C, preferably, from 2 to 4 months at 25 °C. Stable lather (or foam, i.e., a dispersion of gas bubbles in a liquid) means having a 50% wash solution with a volume of at least 218 ml as measured with a SITA Foam Tester R-2000 measurement device as described in the Examples. Substantially free of, as used weight, means less than 1.5%, or less than 1.55% or less than 1.0% or from 0.0001 to 0.5% by weight based on total weight of the wash composition. Substantially free is meant to include no presence (i.e., 0.0% by weight of the composition) of a particular ingredient, like a sulfate based surfactant, as described herein. The wash composition of the present invention, can for example, be substantially free of surfactants that are not an anionic dicarboxylate comprising a glutamate, aspartate or both, and taurate, isethionate, glycinate, amphoteric and/or zwitterionic surfactant. In another embodiment, the wash composition of the present invention, can for example, be substantially free of surfactants that are not an anionic dicarboxylate comprising a glutamate, isethionate and/or taurate and zwitterionic surfactant comprising a betaine or sultaine. The term comprising is meant to encompass the terms consisting essentially of and consisting of. For the avoidance of doubt, and for illustration, the composition of this invention comprising surfactant and water is meant to include a composition consisting essentially of the same and a composition consisting of the same. Except in the operating comparative examples, or where otherwise explicitly indicated, all numbers in this description indicating amounts or ratios of materials or conditions and/or physical properties of materials and/or use are to be understood as modified by the word "about". All ranges presented herein are meant to include all ranges subsumed therein.

### Detailed Description of the Invention

As to the surfactants used in this invention, from 10 to 90% by weight of the surfactants are neutralized, preferably, from 50 to 90%, and most preferably, from 65 to 85% by weight of surfactant is neutralized. Neutralization is achieved with bases that preferably provide as counter ions sodium and potassium ions. It is, however, within the scope of the invention to neutralize surfactant with calcium, magnesium, ammonium or substituted ammonium as counter ions.

Total surfactant used in the wash composition of the present invention is from 1 12%, and most preferably, from 2.25 to 11.5% by weight, based on total weight of the wash composition. In an embodiment of the invention, total surfactant used is from 2.75 to 11% or 3 to 7.25% or 3.35 to 6.95% by weight of the wash composition. The wash composition of the present invention comprises anionic dicarboxylate comprising a glutamate, aspartate or both. Illustrative examples of glutamates suitable for use in the present invention include sodium lauroyl glutamate, sodium cocoyl glutamate, potassium lauroyl glutamate, potassium cocoyl glutamate, sodium or potassium palmitoyl glutamate, mixtures thereof or the like. Illustrative examples of aspartates suitable for use either alone or together with glutamates include sodium lauroyl aspartate, potassium lauroyl aspartate, sodium cocoyl aspartate, potassium cocoyl aspartate, sodium or potassium palmitoyl aspartate, mixtures thereof or the like.

As noted, 40 to 100%, and most preferably, 67.5 to 100% (or in other embodiments 28 to 65% or 38 to 55%) by weight of the total weight of the anionic dicarboxylate present has a fatty acid group, R¹(CR₂)( CR₂)ₘ COO-, that is a linear C₁₆ palmitic acid group. In an embodiment of the invention at least 70% by weight of the total weight of the anionic dicarboxylate present has a fatty acid group, R¹(CR₂)( CR₂)ₘ COO-, that is a linear C₁₆ palmitic acid group. Preferred anionic dicarboxylate, therefore, may include sodium palmitoyl glutamate, potassium palmitoyl glutamate, sodium palmitoyl aspartate, potassium palmitoyl aspartate, any of their C₁₋₃ alkyl substituted derivatives or a mixture thereof. In even other embodiments of the invention, 75 to 100% by weight of the anionic dicarboxylate has a fatty acid group, R¹(CR₂)( CR₂)ₘ COO-, that is a linear C₁₆ palmitic acid group. In still another embodiment, 85 to 100% by weight of the anionic dicarboxylate has a fatty acid group, R¹(CR₂)( CR₂)ₘ COO-, that is a linear C₁₆ palmitic acid group. In yet another embodiment, 90 to 100% by weight of the anionic dicarboxylate has a fatty acid group, R¹(CR₂)( CR₂)ₘ COO-, that is a linear C₁₆ palmitic acid group. In a further embodiment, 100% by weight of the anionic dicarboxylate has a fatty acid group, R¹(CR₂)( CR₂)ₘ COO-, that is a linear C₁₆ palmitic acid group. Preferred anionic dicarboxylate is sodium palmitoyl glutamate, potassium palmitoyl glutamate, or a mixture thereof. Most preferred for use is sodium palmitoyl glutamate.

In an embodiment of the invention, from 0.5 to 7% by weight anionic dicarboxylate comprising a glutamate, aspartate or both is used based on total weight of the wash composition. In another embodiment of the invention, from 0.6 to 5%, and preferably, from 0.8 to 2.5%, and most preferably, from 0.9 to 1.85% by weight anionic dicarboxylate comprising a glutamate, aspartate or both is used based on total weight of the wash composition.

The percent by weight of anionic dicarboxylate comprising a glutamate, aspartate or both that make(s) up the total percent by weight of anionic surfactant in the wash composition preferably is from 5 to 60%; and more preferably, from 6 to 40%, and most preferably, from 7 to 33% (or 7.25 to 16.5%). In another embodiment, from 9.5 to 15% by weight anionic dicarboxylate comprising a glutamate, aspartate or both makes up the total percent by weight of anionic surfactant in the wash composition.

In addition to the anionic dicarboxylate comprising a glutamate, aspartate or mixture thereof used, taurate that may be used is generally identified by the formula:

R²CONR³CH₂CH₂SO₃M

wherein R² is a C₈-C₂₀ alkyl, R³ is a C₁-C₄ alkyl.

M is a solubilizing cation M is such as sodium, potassium, ammonium or substituted ammonium.

As to the isethionate that may be used, these typically include C₈-C₁₈ acyl isethionates. These esters are prepared by a reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

The acyl isethionate may be an alkoxylated isethionate such as is described in Ilardi et al., U.S. Pat. No. 5,393,466, entitled "Fatty Acid Esters of Polyalkoxylated isethonic acid; issued Feb. 28, 1995; hereby incorporated by reference. This compound has the general formula:

R⁴C(O)O-C(X)H--CH₂--(OC(Y)H--CH₂)ₘ--SO₃M

wherein R⁴ is an alkyl group having 8 to 18 carbons, m is an integer from 1 to 4, X and Y are each independently hydrogen or an alkyl group having 1 to 4 carbons and M is a solubilizing cation as previously described.

In an embodiment of the invention, the anionic surfactant can, if desired, further include sodium lauroyl isethionate, sodium cocoyl isethionate, sodium methyl lauroyl taurate, sodium methyl cocoyl taurate or a mixture thereof. Such anionic surfactants are commercially available. Preferred for use is a mixture of sodium methyl lauroyl taurate and sodium lauroyl isethionate and typically at a weight ratio of sodium methyl lauroyl taurate to sodium lauroyl isethionate from 3:1 to 1:3, 2:1 to 1:2, 1.5:1 to 1:1.5, and preferably, 1.35:1 to 1:1.35, and most preferably, 1.15:1 to 1:1.15. In an embodiment of the invention the weight ratio of the same can be 1.1:1 to 1:1.1 or 1:1.

In another embodiment of the invention, and when used, from 0.5 to 8% by weight of anionic surfactant comprising a taurate is included based on total weight of the wash composition. In another embodiment of the invention, from 0.6 to 5%, and preferably, from 1.6 to 3.5%, and most preferably, from 0.9 to 2.5% by weight anionic surfactant comprising a taurate is used based on total weight of the wash composition.

The percent by weight of anionic surfactant comprising a taurate that makes up the total weight of anionic surfactant in the wash composition, when used, is preferably from 5 to 75%; and more preferably, from 6 to 50%, and most preferably, from 7 to 33%. In another embodiment, from 9.5 to 25% (or 9.65 to 20% or 9.75 to 17.5%) by weight anionic surfactant comprising taurate makes up the total percent by weight of anionic surfactant in the wash composition.

In an embodiment of the invention, from 0.5 to 6% by weight isethionate, when used, is included based on total weight of the wash composition. In another embodiment of the invention, from 0.6 to 4%, and preferably, from 0.8 to 2.5%, and most preferably, from 0.9 to 1.75% by weight isethionate is used based on total weight of the wash composition.

When used, the percent by weight of isethionate that makes up the total percent by weight of anionic surfactant in the wash composition typically is from 5 to 40%, or from 5 to 30%, or from 5 to 20%; and preferably, from 6 to 17%, and most preferably, from 7 to 16%. In another embodiment, from 9.5 to 14% percent by weight isethionate makes up the total percent by weight of anionic surfactant in the wash composition.

When both isethionate and taurate are used, collectively they typically make up less than 6.2 %, and preferably from 1.85 to 4.2%, and most preferably, from 2 to 3.75% by weight of the wash composition.

Glycinates are a suitable anionic surfactant that may be used in the wash compositions of the present invention. Illustrative examples include acyl glycinates with lauroyl, myristoyl, behenoyl, palmitoyl, stearoyl, isostearoyl, olivoyl, cocoyl or oleoyl groups. Sodium and/or potassium cocoyl glycinate is often preferred. Other glycinates suitable for use include dihydroxyethyl oleyl glycinate and/or dihydroxyethyl stearyl glycinate.

In an embodiment of the invention, from 0.5 to 6% by weight glycinate may be used based on total weight of the wash composition. In another embodiment of the invention, from 0.6 to 4%, and preferably, from 0.8 to 2.5%, and most preferably, from 0.9 to 1.75% by weight glycinate is used based on total weight of the wash composition.

The percent by weight of glycinate that makes up the total percent by weight of anionic surfactant in the wash composition can be from 5 to 50%; and preferably, from 6 to 33%, and most preferably, from 7 to 25% (or 7.5 to 20% or 8 to 18%) when used. In another embodiment, from 9.5 to 14% by weight glycinate makes up the total percent by weight of anionic surfactant in the wash composition when used.

As to the zwitterionic surfactants suitable for use in the present invention, such surfactants typically include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms generally comply with an overall structural formula:
R⁵--[--C(O)--NH(CH₂)_{q}--]ᵣ--N⁺(R⁶)(R⁷)A--B where R⁵ is alkyl or alkenyl of 7 to 18 carbon atoms;
R⁶ and R⁷ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or --SO₃--.

Suitable zwitterionic surfactants for use in the present invention and within the above general formula include simple betaines of formula:

R⁵--N+(R⁶)(R⁷)-CH₂CO₂-

and amido betaines of formula:
R⁵--CONH(CH₂)ₜ--N⁺--(R⁶)(R⁷)CH₂CO₂⁻where t is 2 or 3.

In both formulae R⁵, R⁶ and R⁷ are as defined previously. R⁵ may, in particular, be a mixture of C₁₂ and C₁₄ alkyl groups derived from coconut oil so that at least half, preferably at least three quarters of the groups R⁵ have 10 to 14 carbon atoms. R⁶ and R⁷ are preferably methyl.

A further possibility is that the zwitterionic surfactant is a sulphobetaine of formula:

R⁵ --N⁺(R⁶)(R⁷)-(CH₂)₃SO₃⁻

or

R⁵--CONH(CH₂)ᵤ --N⁺(R⁶)(R⁷)-(CH₂)₃SO₃⁻

where u is 2 or 3, or variants of these in which --(CH₂)₃SO₃⁻ is replaced by-CH₂C(OH)(H)CH₂SO₃⁻.

In these formulae, R⁵, R⁶ and R⁷ are as previously defined.

Illustrative examples of the zwitterionic surfactants suitable for use include betaines like cocodimethyl carboxymethyl betaine, cocoamidopropyl betaine and laurylamidopropyl betaine. An additional zwitterionic surfactant suitable for use includes cocoamidopropyl sultaine. Such surfactants are made commercially available and it is within the scope of the invention to optionally employ mixtures of the such surfactants. Cocoamidopropyl betaine is often the preferred zwitterionic surfactant used.

Amphoteric surfactants suitable for use in the invention (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for use include sodium lauroamphoacetate, sodium cocoamphoacetate, or mixtures thereof.

As to the amphoteric and/or zwitterionic surfactant used, the same typically makes up from 0.5 to 12%, and preferably, from 2 to 8%, and most preferably, from 3 to 7.5% by weight of the wash composition. In an embodiment of the invention, the amphoteric and/or zwitterionic surfactant used makes up from 4 to 7.25%, preferably from 4.65 to 7.15%, and more preferably, from 5.65 to 7.0% by weight of the wash composition.

In still another embodiment of the invention, the weight ratio of total amphoteric and/or zwitterionic surfactant to total anionic surfactant used in the wash composition is from 1:1 to 3:1, and preferably, from 1.25 :1 to 2.25 :1, and most preferably, from 1.45:1 to 2:1. In yet another embodiment of the invention, the weight ratio of amphoteric and/or zwitterionic surfactant to anionic surfactant used in the wash composition is from 1.48:1 to 1.65:1 or 1.50:1 to 1.60:1.

For the avoidance of doubt, the anionic used in the present invention can be all (100% by weight) glutamate and/or aspartate, and isethionate and/or taurate and the zwitterionic surfactant can be all betaine (100% by weight) whereby the wash composition may have less than 2.5% or 2% or 1% or 0.5% by weight of any other surfactant (or 0.0% by weight of any other surfactant), including cationic, nonionic and any other anionic surfactant including sulfate based surfactant and alpha olefin sulfonate.

As to any optional surfactants that may be used with the surfactant system of this invention, the same include anionic surfactants which can be aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate. The anionic may also be an alkyl sulfate (e.g., C₈-C₁₈ alkyl sulfate) or alkyl ether sulfate (including alkyl glyceryl ether sulfates). Among the alkyl ether sulfates are those having the formula:

R³O(CH₂CH₂O)ₙSO₃M

wherein R⁸ is an alkyl or alkenyl having 8 to 18 carbons, preferably 12 to 18 carbons, n has an average value of at least 1.0, preferably less than 5, and most preferably 1 to 4, and M is as previously defined.

The anionic may optionally include alkyl sulfosuccinates (including mono- and dialkyl, e.g., C₆-C₂₂ sulfosuccinates); alkyl and acyl sarcosinates; sulfoacetates; C₈-C₂₂ alkyl phosphates and phosphonates; alkyl phosphate esters and alkoxyl alkyl phosphate esters, acyl lactates, C₈-C₂₂ monoalkyl succinates and maleates, sulphoacetates, alkyl glucosides mixtures thereof or the like.

Sulfosuccinates suitable for optional use may be monoalkyl sulfosuccinates having the formula:

R⁹OC(O)CH₂CH(SO₃M)CO₂M;

and amide-MEA sulfosuccinates of the formula:
R⁹CONHCH₂CH₂OC(O)CH₂CH(SO₃M)CO₂M wherein R⁹ ranges from C₈-C₂₂ alkyl, where M is as previously defined.

Sarcosinates are generally indicated by the formula:
R¹⁰CON(CH₃)CH₂CO₂M, wherein R¹⁰ ranges from C₈-C₂₀ alkyl and M is as previously defined.

Nonionic surfactants that may optionally be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like.

In an embodiment of the invention optional nonionic surfactants include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH--(CH₂)ₖ(CH₂CH₂O)_{z} H, where i, k are each independently 5 to 15; and z is 5 to 50. In another embodiment of the invention, i and k are each independently 6 to 12; and z is 15 to 35.

The optional nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; which is hereby incorporated by reference or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991; hereby incorporated into the subject application by reference. Cocamide monoethanolamine (cocamide MEA) is a preferred optional nonionic surfactant suitable for use.

Yet other optional nonionic surfactants that may be used in the surfactant system and the end use composition of the present invention include nonionic glucamides like lauroyl methyl glucamide, myristoyl methyl glucamide, cocoyl methyl glucamide, capryloyl/caproyl methyl glucamide, sunfloweroyl methyl glucamide mixtures thereof or the like. When used, nonionic surfactants will make up no more than 1.5% by weight, and preferably, from 0.001 to 1.4%, and most preferably, from 0.01 to 1% by weight of total the total weight of the wash composition.

In still another embodiment of the invention, cationic surfactants may optionally be used.

One class of cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants suitable for use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and stearyl amidopropyl dimethylamine lactate.

Even other useful cationic surfactants suitable for use include quaternized hydrolysates of silk, wheat, and keratin proteins, and it is within the scope of the invention to use mixtures of the aforementioned cationic surfactants.

If optionally used, cationic surfactants will make up no more than 1.0% by weight of the wash composition. When present, they typically make up from 0.01 to 0.7%, and more typically, from 0.1 to 0.5% by weight of the wash composition.

Adjusters suitable to modify/buffer pH may be used. Such pH adjusters include triethylamine, NaOH, KOH, H₂SO₄, HCl, C₆ H₈ O₇ (i.e., citric acid) or mixtures thereof. The pH adjusters are added at amounts such that the resulting pH of the wash composition is from 4.5 to 8, and often, from 5.5 to 7.8, and preferably, from 5.9 to 7.5, and most preferably, from 6 to 6.85 or from 6.8 to 7.2. The pH values may be assessed with commercial instrumentation such as a pH meter made commercially available from Thermo Scientific^{®} and VWR Scientific. Opacifiers like titanium dioxide and/or zinc oxide may also optionally be used. When used, opacifiers make up from 0.01 to 3% by weight of the wash composition.

Optional skin benefit agents suitable for use in this invention are limited only to the extent that they are capable of being topically applied, and able to solubilize in the wash composition at the defined pH.

Illustrative examples of the benefit agents suitable to include in the water portion of the surfactant system and composition are acids, like amino acids, such as arginine, valine or histidine. Additional water soluble benefit agents suitable for use include vitamin B₂, niacinamide (vitamin B₃), vitamin B₆, vitamin C, mixtures thereof or the like. Water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other water soluble benefit agents suitable for use include 4-ethyl resorcinol, extracts like sage, aloe vera, green tea, grapeseed, thyme, chamomile, yarrow, cucumber, liquorice, rosemary extract or mixtures thereof. Water soluble sunscreens like ensulizole may also be used. Total amount of optional water soluble benefit agents (including mixtures) when present in the invention may range from 0.0 to 10%, preferably from 0.001 to 8%, and most preferably, from 0.01 to 6% by weight, based on total weight of the wash composition and including all ranges subsumed therein.

It is also within the scope of the present invention to optionally include oil (i.e., non-water) soluble benefit agents. The only limitation with respect to such oil soluble benefit agents are that the same are suitable to provide a benefit to skin when topically applied.

Illustrative examples of the types of oil soluble benefit agents that may optionally be used in the end use composition of this invention include components like stearic acid, vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol or a mixture thereof. Also, 5-substituted resorcinols like 4-cyclohexyl-5-methylbenzene-1,3-diol, 4-isopropyl-5-methylbenzene-1,3-diol, isobutylamido thiazolyl resorcinol, mixtures thereof or the like may be used. The 5-substituted resorcinols, and their synthesis are described in commonly assigned U.S. Published Patent Application No. 2016/0000669A1.

Even other oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol mixtures thereof or the like. In an embodiment of the invention, the optional oil soluble benefit agent used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred.

Still another retinoic acid precursor suitable for use is hydroxyanasatil retinoate made commercially available under the name Retextra^{®} as supplied by Molecular Design International. The same may be used in a mixture with the oil soluble actives described herein. When optional (i.e., 0.0 to 1.75% by weight) oil soluble active is used in the non-water phase of the wash composition of the invention, it typically makes up from 0.001 to 1.75%, and in another embodiment, from 0.05 to 1.2%, and in yet another embodiment, from 0.1 to 0.5% by weight of the total weight of the wash composition.

Preservatives can desirably be incorporated into the wash composition to protect against the growth of potentially harmful microorganisms. Cosmetic chemists are familiar with appropriate preservatives and routinely choose them to satisfy the preservative challenge test and to provide product stability. Suitable traditional preservatives for use include hydantoin derivatives and propionate salts. Particularly preferred preservatives are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin, benzyl alcohol, methylisothiazolinone, sodium benzoate or mixtures thereof. Other preservatives suitable for use include sodium dehydroacetate, chlorophenesin and decylene glycol. The preservatives should be selected having regard for the use of the composition and possible incompatibilities between the preservatives and other ingredients in the emulsion. Preservatives are preferably employed in amounts ranging from 0.01% to 2% by weight of the total weight of the wash composition. Also preferred is a preservative system with hydroxyacetophenone alone or in a mixture with other preservatives. Even other preferred systems include those using Galguard Lipo G (Galaxy Surfactants), sodium gluconate, sodium benzoate or mixtures thereof.

Conventional emollients like caprylyl glycol, and other conventional alkane diols, may also be used in the wash compositions. When used, emollients make up from 0.1 to 1.75%, and preferably, from 0.2 to 1.25%, and most preferably, from 0.3 to 0.95% by weight of the composition.

Thickening agents (or viscosity builders) are optionally suitable for use in the surfactant systems and end use compositions of the present invention. The preferred thickening agents can be naturally derived or synthetic, are suitable for use in compositions having a pH from 4.5 to 8.0 and are suitable for use in compositions having excellent foaming/lathering qualities. Examples include hydroxypropyl starch phosphate, rheology modifying fatty acids, starches, as well as natural or chemically modified polysaccharides. Polysaccharides suitable for use include celluloses; microcrystalline celluloses; hemicelluloses; cellulose gums; pectins, like homogalacturonan, xylogalacturonan, rhamnogalacturonan; exudate polysaccharide gums, like gum arabic, gum tragacanth, acacia gum, karaya gum, ghatti gum; extractive polysaccharide gums, like Konjac gum, guar gum, locust bean gum, mustard mucilage gum, flaxseed mucilage gum, okra mucilage gum, psyllium gum; seaweed polysaccharides, like carrageenan, agar, or alginate; microbial polysaccharides, like xanthan gum, gellan gum, or pullulan gum; fermentation polysaccharides, like sclerotium gums; diutan gum, fructans, like inulin or levan; or mixtures of thereof. Other thickening agents suitable for use include acids like capric, lauric, stearic, myristic, or palmitic acid or mixtures thereof. Still others include acrylate copolymers, polymeric viscosity aids like esters of polyalkoxylated polyols and fatty acids. Examples of such thickeners include Rheocare^{™} TTA from BASF, Hostagel^{®} AV from Clariant, Aculyn^{™} from Dow Chemical, Carbopol^{®} made commercially available by Lubrizol, PEG 18 glyceryloleate/cocoate, polyethylene glycol 6000 distearate, INCl name of PEG-150 distearate; PEG 120 methyl glucose dioleate and PEG 120 methylglucose trioleate (Glucomate^{™} DOE-120 and Glucomate^{™} VLT made available by Lubrizol); PEG-150 Pentaerythrityl Tetrastearate (Crothix^{™}, Crothix^{™} Liquid, and Versathix^{™} made available by Croda); PEG-150 Polyglyceryl-2 Tristerate (Genapol^{®} LT made available by Clariant); PEG/PPG-120/10-Trimethlolpropane Trioleate (Arlypon^{®} TT made available by BASF).

Typical amounts may range from 0.0 to 12%, and often, from 0.0 to 8%, and preferably, 0.001 to 6%, and most preferably, from 0.01 to 5% by weight of the wash composition. In an embodiment of the invention, thickening agent can make up from 0.02 to 1% by weight of the wash composition.

Fragrances, fixatives, chelators (like EDTA) salts (like NaCl) and exfoliants may optionally be included in the wash composition of the present invention. Each of these substances may range from about 0.03 to about 5%, preferably between 0.1 and 3% by weight of the wash composition. To the extent the exfoliants are used, those selected should be of small enough particle size so that they do not impede the performance of wash composition or any packaging it is dispensed from.

Conventional emulsifiers having an HLB of greater than 8 may optionally be used. Illustrative examples include Tween, 40, 60, 80, polysorbate 20 and mixtures thereof. Typically, emulsifiers for water continuous systems, when used, make up from 0.3 to 2.5% by weight of the wash composition.

Conventional humectants may optionally be employed as additives in the present invention to assist in moisturizing skin when such emulsions are topically applied. These are generally polyhydric alcohol type materials. Typical polyhydric alcohols include glycerol (i.e., glycerine or glycerin), propylene glycol, dipropylene glycol, polypropylene glycol (e.g., PPG-9), polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.0 to 35% by weight of the total weight of the liquid and composition. Often, humectant makes up from 0.0 to 20%, and preferably, from 0.001 to 15% by weight (most preferably, from 2 to 12% by weight) of the total weight of the wash composition.

Lamellar liquid wash may be induced with a structurant that is preferably a fatty acid. More preferably, the structurant is selected from the group consisting of caprylic acid, lauric acid, myristic acid or a mixture thereof. Typically, structurant makes up from 1 to 12%, and preferably, from 2 to 10%, and most preferably, from 3 to 8.5% by weight of the wash composition.

The wash composition of the present invention can, and preferably, does comprise less than 3% by weight soap (e.g., C₁₄- C₁₈ fatty acid soap), and preferably less than 1.5% by weight soap, and most preferably, no soap.

When making the wash compositions of this invention, the desired ingredients may be mixed with conventional apparatus under moderate shear atmospheric conditions, with temperature being from 25°C to 80°C. Such wash compositions will typically have a viscosity from 1.0 to 25,000 cps (500 to 20,000 mPa-s), and preferably, from 500 to 20,000 cps (500 to 20,000 mPa-s), and most preferably, from 1000 to 13,500 cps (1000 to 13,500 mPa-s). In an embodiment of the invention, the viscosity of the wash composition is from 3,250 to 9,500 cps (3,250 to 9,500 mPa-s).

The packaging for the wash composition of this invention can be a pump dispenser, tube, jar or plastic bottle. Preferably, the package is a bottle which is made with recyclable materials and/or post-consumer resins.

The following examples are provided to facilitate an understanding of the present invention. They are not intended to limit the scope of the claims.

### Examples

The following base wash composition was made by mixing ingredients as identified in Table I with pressure at atmospheric and temperature at 25 to 75°C. The pH was targeted from 6.8 to 7.2.

**TABLE I**

| Ingredient | Weight Percent |
|---|---|
| Surfactant | Per Example 1 |
| Cocamide MEA | 0.5 |
| Lauric acid | 0.25 |
| Stearic acid | 0.31 |
| Thickener | 1.0 |
| Glycerol | 5.0 |
| Opacifier | 1.0 |
| NaOH | 0.25 |
| Citric Acid | To target pH |
| NaCl | 0.1 |
| Water | To Balance |

### Example I

Surfactants added to the base composition above are as identified in weight percent of the base composition.

### CONTROL SURFACTANTS

sodium lauroyl isethionate (2.1%); sodium methyl lauroyl taurate (2.1%); cocamidopropyl betaine (6.5%)

### INVENTIVE REDUCED PALM KERNEL OIL SURFACTANTS

sodium lauroyl isethionate (1.4%); sodium methyl lauroyl taurate (1.4%); sodium palmitoyl glutamate (1.4%); cocamidopropyl betaine (6.5%)

Foam volume (in ml) as shown in the data of Table II, was measured with a SITA Foam Tester R-2000 measurement device, with temperature set at 40°C and the device set at automated. Compositions made according to the invention generated equivalent amounts of lather when compared to compositions made with control surfactants.

The wash compositions made according to the present invention were stored for 4 months at 25 °C. Surprisingly and with reduced levels of palm kernel oil derived surfactants, the wash compositions were stable, free of syneresis, and color and odour changes as determined by trained panelists. Moreover, after washing with compositions consistent with the invention, the panelists concluded that the wash compositions lather well, rinse easily and produce a soft and smooth sensation after use.

**Table II**

| WASH AGITATION IN SECONDS | CONTROL SURFACTANTS | | REDUCED PALM KERNEL OIL SURFACTANTS | |
|---|---|---|---|---|
| | Foam Volume, ml | Standard deviation | Foam Volume, ml | Standard Deviation |
| 15 | 228 | 11.6 | 225 | 7.7 |
| 30 | 311 | 28.2 | 341 | 19.7 |
| 45 | 404 | 32 | 418 | 12.5 |
| 60 | 426 | 28.7 | 438 | 15.3 |

### Example II

The CIM (colorimetric index of mildness) of the wash composition was determined by the Corneosurfametry *ex-vivo* test method at the standard test condition with the procedure outlined in the technical publication, "Modified Corneosurfametry as a new accelerated high-throughput ex-vivo methodology for predicting cleanser effects towards human skin" by Liu et al., International Journal of Cosmetic Science, Vol. 38, pg. 178-186, 2016.

**Table III**

| Wash Composition | Colorimetric Index of Mildness (CIM) |
|---|---|
| Water | 74.9 |
| Control Surfactant | 70.6 |
| Reduced Palm Kernel Oil Surfactants | 74.2 |

The data in Table III unexpectedly shows that when reduced palm kernel oil surfactants according to the invention are used in base compositions, the wash compositions are significantly milder and similar to the mildness of water.

### Example III

**Table IV**

| Surfactants* (SLI, SMLT, SPG, CAPB) (Weight Percent) | Thickening System (Weight Percent, respectively) | Viscosity, mPas | Stability |
|---|---|---|---|
| 1.4, 1.4, 1.4, 6.5 | Starch/Cellulose* 2.9,0.37 | 22,700 | Stable |
| 1.05, 2.1, 1.05, 6.5 | Starch/Cellulose* 2.9,0.37 | 7,011 | Stable |
| 1.4,1.4, 1.4, 6.5 | Carbapol 980/Versathix 0.5, 0.56 | 12,690 | Stable |
| 2.1, 2.1, 0, 6.5 | Carbapol 980/Versathix 0.5, 0.56 | 8,450 | Stable |
| 0, 2.7, 2.7, 5.4 | Carbapol 980/Versathix 0.5, 0.56 | 15,330 | Stable |
| 2.7, 2.7, 2.7, 2.7 | Carbapol 980/Versathix 0.5, 0.56 | 10,670 | Stable |
| 4.05, 0, 4.05, 2.7 | Carbapol 980/Versathix 0.5, 0.56 | 7,529 | Stable |
| 0, 4.5, 4.05, 2.7 | Carbapol 980/Versathix 1.0, 0.61 | 7,286 | Stable |
| 2.7, 0, 2.7, 5.4 | Carbapol 980/Versathix 0.35, 0.39 | 8,378 | Stable |
| 1.8, 1.8, 1.8, 5.4 | Carbapol 980/Versathix 0.35, 0.39 | 6,489 | Stable |

| | | | |
|---|---|---|---|
| *Surfactants: sodium lauroyl isethionate (SLI), sodium methyl lauroyl taurate (SMLT), sodium palmitoyl glutamate (SPG), cocoamidopropyl betaine (CAPB) in weight percent, respectively. *Hydroxypropyl Starch Phosphate/microcrystalline cellulose | | | |

The data in Table IV unexpectedly shows that wash compositions containing sodium palmitoyl glutamate in accordance with the invention can be stabilized using natural polymer systems such as starch and cellulose as well as synthetic thickening systems consisting of Carbapol 980 and Versathix. The stability of the compositions with sodium palmitoyl glutamate were surprisingly consistent with wash compositions having traditional surfactants derived from palm kernel oil.

## Claims

1. A wash composition comprising:
a) an anionic dicarboxylate comprising a glutamate, aspartate or both, the glutamate and/or aspartate having a formula: 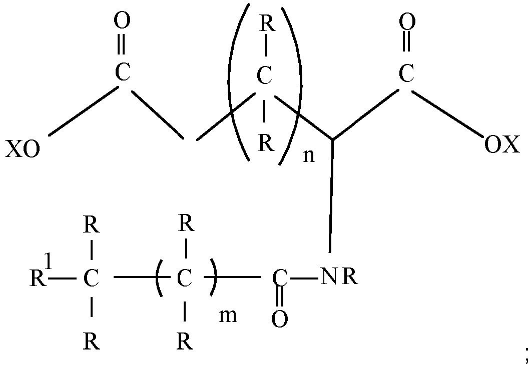
b) an amphoteric or zwitterionic surfactant or both; and
c) water
wherein total surfactant used is from 1 to 12% by weight of the composition,
wherein nonionic surfactants will make up no more than 1.5 wt%, by weight of the wash composition,
wherein the composition has a pH from 4.5 to 8.0,
1.5 to 90%, and preferably, 1.75 to 80.0%, and most preferably, from 2 to 70% by weight of total weight surfactant being anionic surfactant and further
wherein from 3.3 to 100%, and preferably, from 3.3 to 80%, and most preferably, from 10.0 to 45% by weight of the total weight anionic surfactant is the anionic dicarboxylate with the proviso that from 10 to 95% by weight of surfactant in the composition is neutralized, where:
i) each R is independently an H or a C₁₋₃ alkyl, and where at least 50% of the R groups are H, and R¹ is a C₈-C₂₂ alkyl group with the further proviso that at least 40 to 100%, most preferably, 67.5 to 100% by weight of the total weight of the anionic dicarboxylate present has a fatty acid group, R¹(CR₂)( CR₂)ₘ COO-, that is a linear C₁₆ palmitic acid group;
ii) each X is independently H⁺, Na⁺, K⁺, Ca⁺ or Mg⁺, where each X is not simultaneously H⁺; and
iii) n is 0 or 1, and m is an integer from 0 to 3,
the composition having at least 10 to 55% of all surfactant free of a hydrophobic group derived from palm kernel oil.

2. The wash composition according to claim 1, further comprising at least one anionic surfactant which is a taurate, isethionate or glycinate.

3. The wash composition according to claim 2, comprising:
a) an anionic dicarboxylate comprising a glutamate, aspartate or both, the glutamate and/or aspartate having a formula: 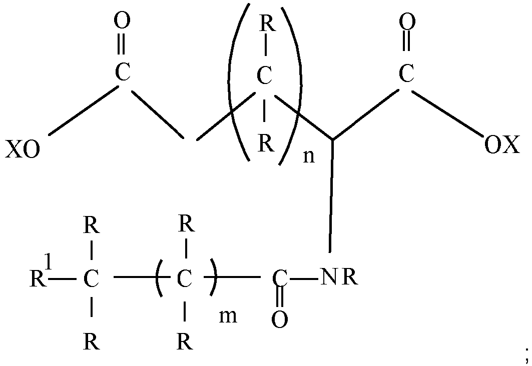
b) anionic surfactant comprising a taurate and an isethionate;
c) at least one surfactant which is a zwitterionic surfactant; and
d) water
wherein total surfactant used is from 1 to 12% by weight of the composition,
wherein nonionic surfactants will make up no more than 1.5 wt%, by weight of the wash composition,
wherein the composition has a pH from 4.5 to 8.0, 1.5 to 90%, and preferably, 1.75 to 80.0% , and most preferably, from 2 to 70% by weight of total weight surfactant being anionic surfactant a+b and further wherein from 3.3 to 90%, and preferably, from 3.3 to 80%, and most preferably, from 10.0 to 45% by weight of the total weight anionic surfactant a+b is the anionic dicarboxylate with the proviso that from 10 to 95% by weight of surfactant in the composition is neutralized, where:
i) each R is independently an H or a C₁₋₃ alkyl, and where at least 50% of the R groups are H, and R¹ is a C₈-C₂₂ alkyl group with the further proviso that at least 40 to 100%, and most preferably, 67.5 to 100% by weight of the total weight of the anionic dicarboxylate present has a fatty acid group, R¹(CR₂)( CR₂)ₘ COO-, that is a linear C₁₆ palmitic acid group;
ii) each X is independently H⁺, Na⁺, K⁺, Ca⁺ or Mg⁺, where each X is not simultaneously H⁺; and
iii) n is 0 or 1, and m is an integer from 0 to 3,
the composition comprising:
I. from 0.5 to 7% by weight anionic dicarboxylate comprising a glutamate, aspartate or both;
II. from 0.5 to 6% by weight isethionate making up from 5 to 40% by weight of the total weight percent of anionic surfactant;
III. from 0.5 to 8% by weight taurate making up from 5 to 70% by weight of the total weight percent of anionic surfactant; and
IV. 0.5 to 8% by weight zwitterionic surfactant comprising a betaine
the composition having at least 10 to 55% of all surfactant free of a hydrophobic group derived from palm kernel oil.

4. The composition according to claim 3, wherein the anionic dicarboxylate comprising glutamate, aspartate or both makes up from 5 to 60% by weight of the total weight percent of anionic surfactant.

5. The composition according to any one of the preceding claims, wherein each R is independently an H or a C₁₋₃ alkyl, and where at least 50% of the R groups are H, and R¹ is a C₈-C₂₂ alkyl group with the further proviso that 67.5 to 100% by weight of the total weight of the anionic dicarboxylate present has a fatty acid group, R¹(CR₂)( CR₂)ₘ COO-, that is a linear C₁₆ palmitic acid group.

6. The composition according to any one of the preceding claims , wherein total surfactant used is from 2 to 12% and total anionic surfactant is from 1.8 to 8% by weight of the composition.

7. The composition according to any of the preceding claims wherein the composition has a pH from 5.5 to 7.8.

8. The composition according to any of the preceding claims wherein the composition further comprises 12-hydroxystearic acid.

9. The composition according to any of the preceding claims wherein from 15 to 45% of all surfactant in the wash composition is free of a hydrophobic group derived from palm kernel oil.

10. The composition according to any of the preceding claims wherein from 20 to 35% of all surfactant in the wash composition is free of a hydrophobic group derived from palm kernel oil.

11. The composition according to any of the preceding claims wherein the composition comprises less than 1.5 % by weight, based on total weight of the wash composition, of a sulfate-based surfactant.

12. The composition according to any of the preceding claims wherein from 25 to 55%, and preferably, from 35 to 45% by weight of total surfactant is anionic surfactant and from 15 to 40%, and preferably, from 20 to 30%, and most preferably, from 22 to 28% by weight of the total weight anionic surfactant is the anionic dicarboxylate.

13. The composition according to any of the preceding claims wherein from 50 to 90%, and preferably, 65 to 85% by weight of surfactant in the composition is neutralized.

14. A method for cleaning a surface comprising the steps of:
a) contacting a surface with the wash composition according to any one of the preceding claims; and
a) shearing the wash composition on the surface;
b) rinsing and/or wiping the wash composition off the surface with water.

## Patentansprüche

1. Waschmittelzusammensetzung, umfassend:
a) ein anionisches Dicarboxylat, das ein Glutamat, Aspartat oder beides umfasst, wobei das Glutamat und/oder Aspartat die Formel 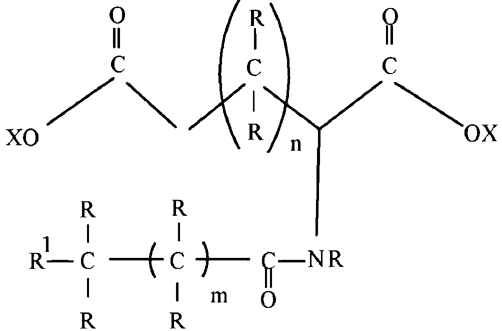 aufweisen;
b) ein amphoteres oder zwitterionisches Tensid oder beides; und
c) Wasser,
wobei die Gesamtmenge an verwendetem Tensid 1 bis 12 Gew.-% der Zusammensetzung beträgt,
wobei nichtionische Tenside nicht mehr als 1,5 Gew.-% nach Gewicht der Waschmittelzusammensetzung ausmachen,
wobei die Zusammensetzung einen pH-Wert von 4,5 bis 8,0 aufweist, 1,5 bis 90% und vorzugsweise 1,75 bis 80,0% und höchst bevorzugt 2 bis 70 Gew.-% des Gesamtgewichts des Tensids ein anionisches Tensid sind und
wobei ferner 3,3 bis 100%, vorzugsweise 3,3 bis 80% und höchst bevorzugt 10,0 bis 45 Gew.-% des Gesamtgewichts des anionischen Tensids ein anionisches Dicarboxylat ist, dies mit der Maßgabe, dass 10 bis 95 Gew.-% des Tensids in der Zusammensetzung neutralisiert sind, wobei:
i) jedes R unabhängig voneinander H oder ein C₁₋₃-Alkyl ist und wobei mindestens 50% der R-Gruppen H sind, und R¹ eine C₈-C₂₂-Alkylgruppe ist, dies mit der weiteren Maßgabe, dass mindestens 40 bis 100% Gew.- %, höchst bevorzugt 67,5 bis 100 Gew.-% des Gesamtgewichts des vorhandenen anionischen Dicarboxylats eine Fettsäuregruppe aufweisen, R¹(CR₂)(CR₂)ₘCOO⁻, das heißt, eine lineare C₁₆-Palmitinsäuregruppe ist;
ii) jedes X unabhängig voneinander H⁺, Na⁺, K⁺, Ca⁺ oder Mg⁺ ist, wobei jedes X nicht gleichzeitig H⁺ ist; und
iii) n 0 oder 1 ist und m eine ganze Zahl von 0 bis 3 ist,
wobei die Zusammensetzung mindestens 10 bis 55% aller Tenside enthält, die frei von einer hydrophoben Gruppe sind, gewonnen aus Palmkernöl.

2. Waschmittelzusammensetzung nach Anspruch 1, die ferner mindestens ein anionisches Tensid umfasst, das ein Taurat, Isethionat oder Glycinat ist.

3. Waschmittelzusammensetzung nach Anspruch 2, umfassend:
a) ein anionisches Dicarboxylat, das ein Glutamat, Aspartat oder beides umfasst,
wobei das Glutamat und/oder Aspartat die Formel 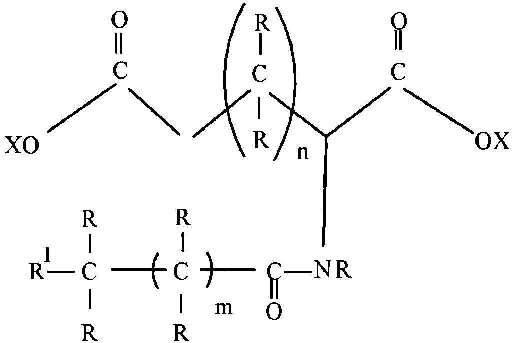 aufweisen;
b) ein anionisches Tensid, das ein Taurat und ein Isethionat umfasst;
c) mindestens ein Tensid, das ein zwitterionisches Tensid ist; und
d) Wasser;
wobei die Gesamtmenge an verwendetem Tensid 1 bis 12 Gew.-% der Zusammensetzung beträgt,
wobei nichtionische Tenside nicht mehr als 1,5 Gew.-% nach Gewicht der Waschmittelzusammensetzung ausmachen,
wobei die Zusammensetzung einen pH-Wert von 4,5 bis 8,0 aufweist, 1,5 bis 90% und vorzugsweise 1,75 bis 80,0% und höchst bevorzugt 2 bis 70 Gew.-% des Gesamtgewichts des Tensids ein anionisches Tensid a+b sind, und wobei ferner 3,3 bis 90%, vorzugsweise 3,3 bis 80% und höchst bevorzugt 10,0 bis 45 Gew.-% des Gesamtgewichts des anionischen Tensids a+b das anionische Dicarboxylat sind, dies mit der Maßgabe, dass 10 bis 95 Gew.-% des Tensids in der Zusammensetzung neutralisiert sind, wobei:
i) jedes R unabhängig voneinander H oder ein C₁₋₃-Alkyl ist, wobei mindestens 50% der R-Gruppen H sind und R¹ eine C₈-C₂₂-Alkylgruppe ist, dies mit der weiteren Maßgabe, dass mindestens 40 bis 100% und höchst bevorzugt 67,5 bis 100 Gew.-% des Gesamtgewichts des vorhandenen anionischen Dicarboxylats eine Fettsäuregruppe, R¹(CR₂)(CR₂)ₘCOO-, aufweisen, die eine lineare C₁₆-Palmitinsäuregruppe ist;
ii) jedes X unabhängig voneinander H⁺, Na⁺, K⁺, Ca⁺ oder Mg⁺ ist, wobei jedes X nicht gleichzeitig H⁺ ist; und
iii) n 0 oder 1 ist und m eine ganze Zahl von 0 bis 3 ist,
wobei die Zusammensetzung umfasst:
I. 0,5 bis 7 Gew.-% anionisches Dicarboxylat, das ein Glutamat, Aspartat oder beides umfasst;
II. 0,5 bis 6 Gew.-% Isethionat, das 5 bis 40 Gew.-% des Gewichtsanteils des anionischen Tensids ausmacht;
III. 0,5 bis 8 Gew.-% Taurat, das 5 bis 70 Gew.-% des Gewichtsanteils des anionischen Tensids ausmacht; und
IV. 0,5 bis 8 Gew.-% zwitterionisches Tensid, das ein Betain umfasst,
wobei die Zusammensetzung mindestens 10 bis 55% aller Tenside frei von einer hydrophoben Gruppe, gewonnen aus Palmkernöl, enthält.

4. Zusammensetzung nach Anspruch 3, wobei das anionische Dicarboxylat, das Glutamat, Aspartat oder beides umfasst, 5 bis 60 Gew.-% des Gewichtsanteils des anionischen Tensids ausmacht.

5. Zusammensetzung nach irgendeinem der vorhergehenden Ansprüche, wobei jedes R unabhängig voneinander H oder C₁₋₃-Alkyl ist und wobei mindestens 50% der R-Gruppen H sind und R¹ eine C₈-C₂₂-Alkylgruppe ist, dies mit der weiteren Maßgabe, dass 67,5 bis 100 Gew.-% des Gesamtgewichts des vorhandenen anionischen Dicarboxylats eine Fettsäuregruppe, R¹(CR₂)(CR₂)ₘCOO⁻, aufweisen, die eine lineare C₁₆-Palmitinsäuregruppe ist.

6. Zusammensetzung nach irgendeinem der vorhergehenden Ansprüche, wobei die Gesamtmenge an Tensiden 2 bis 12% und die Gesamtmenge an anionischem Tensid 1,8 bis 8 Gew.-% der Zusammensetzung beträgt.

7. Zusammensetzung nach einem der vorhergehenden Ansprüche, wobei die Zusammensetzung einen pH-Wert von 5,5 bis 7,8 aufweist.

8. Zusammensetzung nach einem der vorhergehenden Ansprüche, wobei die Zusammensetzung ferner 12-Hydroxystearinsäure umfasst.

9. Zusammensetzung nach einem der vorhergehenden Ansprüche, wobei 15 bis 45% aller Tenside in der Waschmittelzusammensetzung frei von einer hydrophoben Gruppe sind, gewonnen aus Palmkernöl.

10. Zusammensetzung nach einem der vorhergehenden Ansprüche, wobei 20 bis 35% aller Tenside in der Waschmittelzusammensetzung frei von einer hydrophoben Gruppe sind, gewonnen aus Palmkernöl.

11. Zusammensetzung nach einem der vorhergehenden Ansprüche, wobei die Zusammensetzung weniger als 1,5 Gew.-%, bezogen auf das Gesamtgewicht der Waschmittelzusammensetzung, eines Sulfat-basierten Tensids umfasst.

12. Zusammensetzung nach einem der vorhergehenden Ansprüche, wobei 25 bis 55% und vorzugsweise 35 bis 45% des gesamten Tensids ein anionisches Tensid sind und 15 bis 40% und vorzugsweise 20 bis 30% und höchst bevorzugt 22 bis 28 Gew.-% des Gesamtgewichts des anionischen Tensids das anionische Dicarboxylat sind.

13. Zusammensetzung nach einem der vorhergehenden Ansprüche, wobei 50 bis 90% und vorzugsweise 65 bis 85 Gew.-% des Tensids in der Zusammensetzung neutralisiert sind.

14. Verfahren zum Reinigen einer Oberfläche, umfassend die folgenden Schritte:
a) Inkontaktbringen einer Oberfläche mit der Waschmittelzusammensetzung nach einem der vorhergehenden Ansprüche; und
a) Scherung der Waschmittelzusammensetzung auf der Oberfläche;
b) Abspülen und/oder Abwischen der Waschmittelzusammensetzung von der Oberfläche mit Wasser.

## Revendications

1. Composition de lavage comprenant :
a) un dicarboxylate anionique comprenant un glutamate, de l'aspartate ou les deux, le glutamate et/ou l'aspartate ayant une formule : 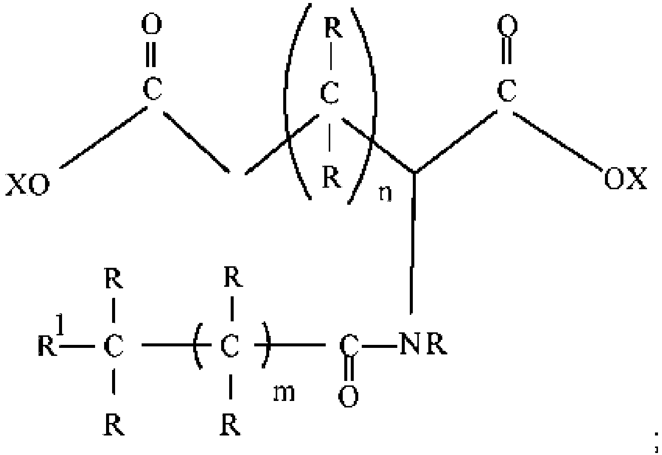
b) un tensioactif amphotérique ou zwitterionique ou les deux ; et
c) de l'eau
dans laquelle la quantité totale de tensioactif utilisée est comprise entre 1 et 12 % en poids de la composition,
dans laquelle les tensioactifs non ioniques ne constituent pas plus de 1,5 % en poids de la composition de lavage,
dans laquelle la composition présente un pH compris entre 4,5 et 8,
1,5 à 90 %, et de préférence 1,75 à 80 %, et de préférence 2 à 70 % en poids du poids total de tensioactif étant un tensioactif anionique,
et dans laquelle, en outre, 3,3 à 100 %, et de préférence 3,3 à 80 %, et de préférence 10 à 45 % en poids du poids total de tensioactif anionique sont le dicarboxylate anionique à condition que 10 à 95 % en poids de tensioactif dans la composition soient neutralisés, où :
i) chaque R est indépendamment un H ou un alkyle en C₁₋₃, et où au moins 50 % des groupes R sont H, et R¹ est un groupe alkyle en C₈-C₂₂ à condition en outre qu'au moins 40 à 100 %, de préférence 67,5 à 100 % en poids du poids total du dicarboxylate anionique présent présente un groupe d'acide gras, R¹(CR₂)(CR₂)ₘ COO-, qui soit un groupe d'acide palmitique en C₁₆ linéaire ;
ii) chaque X est indépendamment H⁺, Na⁺, K⁺, Ca⁺ ou Mg⁺, où chaque X n'est pas simultanément H⁺ ; et
iii) n est 0 ou 1, et m est un entier compris entre 0 et 3,
la composition ayant au moins 10 à 55 % de tous les tensioactifs exempts d'un groupe hydrophobe dérivé d'huile de palmiste.

2. Composition de lavage selon la revendication 1, comprenant en outre au moins un tensioactif anionique qui est un taurate, un iséthionate ou un glycinate.

3. Composition de lavage selon la revendication 2, comprenant :
a) un dicarboxylate anionique comprenant un glutamate, de l'aspartate ou les deux, le glutamate et/ou l'aspartate ayant une formule : 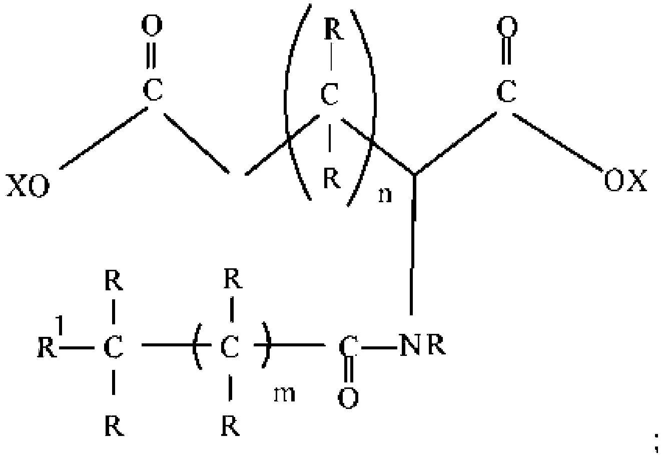
b) un tensioactif anionique comprenant un taurate et un iséthionate ;
c) au moins un tensioactif qui est un tensioactif zwitterionique ; et
d) de l'eau
dans laquelle la quantité totale de tensioactif est comprise entre 1 et 12 % en poids de la composition,
dans laquelle les tensioactifs non ioniques ne constituent pas plus 1,5 % en poids de la composition de lavage,
dans laquelle la composition présente un pH compris entre 4,5 et 8, 1,5 à 90 %, et de préférence 1,75 à 80 %, et de préférence 2 à 70 % en poids du poids total de tensioactif étant un tensioactif anionique a+b, et dans laquelle, en outre, 3,3 à 90 %, et de préférence 3,3 à 80 %, et de préférence 10 à 45 % en poids du poids total de tensioactif anionique a+b sont le dicarboxylate anionique à condition que 10 à 95 % en poids du tensioactif dans la composition soient neutralisés, où :
i) chaque R est indépendamment un H ou un alkyle en C₁₋₃, et où au moins 50 % des groupes R sont H, et R¹ est un groupe alkyle en C₈-C₂₂ à condition en outre qu'au moins 40 à 100 %, et de préférence 67,5 à 100 % en poids du poids total du dicarboxylate anionique présent présentent un groupe d'acide gras, R¹(CR₂)(CR₂)ₘ COO-, qui soit un groupe d'acide palmitique en C₁₆ linéaire ;
ii) chaque X est indépendamment H⁺, Na⁺, K⁺, Ca⁺ ou Mg⁺, où chaque X n'est pas simultanément H⁺ ; et
iii) n est 0 ou 1, et m est un entier compris entre 0 et 3,
la composition comprenant :
I. 0,5 à 7 % en poids de dicarboxylate anionique comprenant un glutamate, de l'aspartate ou les deux ;
II. 0,5 à 6 % en poids d'iséthionate constituant 5 à 40 % en poids du pourcentage en poids total de tensioactif anionique ;
III. 0,5 à 8 % en poids de taurate constituant 5 à 70 % en poids du pourcentage en poids total de tensioactif anionique ; et
IV. 0,5 à 8 % en poids de tensioactif zwitterionique comprenant une bétaïne,
la composition ayant au moins 10 à 55 % de tous les tensioactifs exempts d'un groupe hydrophobe dérivé de l'huile de palmiste.

4. Composition selon la revendication 3, dans laquelle le dicarboxylate anionique comprenant un glutamate, de l'aspartate ou les deux constitue 5 à 60 % en poids du pourcentage en poids total de tensioactif anionique.

5. Composition selon l'une quelconque des revendications précédentes, dans laquelle chaque R est indépendamment un H ou un alkyle en C₁₋₃, et dans laquelle au moins 50 % des groupes R sont H, et R¹ est un groupe alkyle en C₈-C₂₂ à condition en outre que 67,5 à 100 % en poids du poids total du dicarboxylate anionique présent présentent un groupe d'acide gras, R¹(CR₂)(CR₂)ₘ COO-, qui soit un groupe d'acide palmitique en C₁₆ linéaire.

6. Composition selon l'une quelconque des revendications précédentes, dans laquelle la quantité totale de tensioactif utilisée est comprise entre 2 et 12 % et la quantité totale de tensioactif anionique est comprise entre 1,8 et 8 % en poids de la composition.

7. Composition selon l'une quelconque des revendications précédentes, dans laquelle la composition présente un pH compris entre 5,5 et 7,8.

8. Composition selon l'une quelconque des revendications précédentes, dans laquelle la composition comprend en outre de l'acide 12-hydroxystéarique.

9. Composition selon l'une quelconque des revendications précédentes, dans laquelle 15 à 45 % de tous les tensioactifs présents dans la composition de lavage sont exempts d'un groupe hydrophobe dérivé d'huile de palmiste.

10. Composition selon l'une quelconque des revendications précédentes, dans laquelle 20 à 35 % de tous les tensioactifs présents dans la composition de lavage sont exempts d'un groupe hydrophobe dérivé de l'huile de palmiste.

11. Composition selon l'une quelconque des revendications précédentes, dans laquelle la composition comprend moins de 1,5 en poids, sur la base du poids total de la composition de lavage, d'un tensioactif à base de sulfates.

12. Composition selon l'une quelconque des revendications précédentes, dans laquelle 25 à 55 %, et de préférence 35 à 45 % en poids de la quantité totale de tensioactif sont un tensioactif anionique et 15 à 40 %, et de préférence 20 à 30 %, et de préférence 22 à 28 % en poids du poids total de tensioactif anionique sont le dicarboxylate anionique.

13. Composition selon l'une quelconque des revendications précédentes, dans laquelle 50 à 90 %, et de préférence 65 à 85 % en poids du tensioactif présent dans la composition sont neutralisés.

14. Procédé de nettoyage d'une surface comprenant les étapes consistant à :
a) mettre en contact une surface avec la composition de lavage selon l'une quelconque des revendications précédentes ; et
b) cisailler la composition de lavage sur la surface ;
c) rincer et/ou essuyer la composition de lavage sur la surface avec de l'eau.
